# EUROPEAN PATENT APPLICATION

(11) **EP 4 201 423 A1**
(43) Date of publication of application: **28.06.2023**
(21) Application number: 21786322.4
(22) Date of filing: 05.08.2021
(51) Int. Cl.: A61K 39/095, A61K 39/215, A61P 31/00, A61P 31/14, A61P 37/04, C07K 14/005, C07K 14/165, C07K 14/22

(54) **COMPOSITIONS OF SARS-COV-2 VACCINES BASED ON THE RECEPTOR BINDING DOMAIN, EXPRESSED AS A DIMER, AND THE OUTER MEMBRANE VESICLE OF MENINGOCOCCAL GROUP B BACTERIA**

(30) Priority: 20.08.2020 CU 20200057
(71) Applicant: INSTITUTO FINLAY DE VACUNAS, La Habana 11300 (CU); Centro de Inmunologia Molecular, Playa, La Habana 11300 (CU)
(72) Inventor: VALDÉS BALBÍN, Yury, Playa La Habana 11300 (CU); VEREZ BENCOMO, Vicente Guillermo, Playa La Habana 11300 (CU); GARCÍA RIVERA, Dagmar, La Habana 17100 (CU); CLIMENT RUIZ, Yanet, La Habana 10400 (CU); FERNÁNDEZ CASTILLO, Sonsire, Playa La Habana 11300 (CU); RODRÍGUEZ NODA, Laura Marta, La Habana 10400 (CU); GONZÁLEZ RODRÍGUEZ, Humberto, La Habana 11500 (CU); RAMÍREZ GONZÁLEZ, Ubel Jesús, La Habana 17100 (CU); CHOVEL CUERVO, Mario Landys, La Habana 17100 (CU); PÉREZ NICADO, Rocmira, La Habana (CU); SÁNCHEZ RAMÍREZ, Belinda, La Habana 11300 (CU); OJITO MAGAZ, Eduardo, La Habana 11300 (CU); BOGGIANO AYO, Tammy, La Habana 11300 (CU); OLIVA HERNÁNDEZ, Reynaldo, Playa La Habana 11300 (CU); CHEN, Guang Wu, Chengdu 610041 (CN)
(74) Representative: V.O.
(86) International application number: PCT/CU2021/050007
(87) International publication number: WO 2022/037727

(57) **Abstract**

This invention is related to biotechnology; in particular, to the field of human health. The described vaccine compositions induce a neutralizing immune response against the SARS-CoV-2 virus. These compositions include a portion of the receptor binding protein of the SARS-Cov-2 virus, as antigen, outer-membrane vesicles of the *Neisseria meningitides* group B bacteria, as immunopotentiating component, and an adjuvant. The vaccine compositions that are described in this invention are useful in the prevention of infection with the SARS-CoV-2 virus.

## Description

### TECHNICAL FIELD

This invention pertains to the field of biotechnology; in particular, the vaccines for the prevention of COVID-19. Specifically, the invention describes new vaccine compositions that are designed to prevent infections with the SARS-CoV-2 virus. These compositions comprise the receptor binding domain ("RBD") of the SARS-CoV-2 virus as relevant antigen that is expressed as a stable dimer, as well as outer membrane vesicles ("OMV") of the *Neisseria meningitidis* group B bacteria, and an adjuvant. Such a combination can induce high antibody titers and a cellular response against the SARS-CoV-2 virus.

### TECHNOLOGY BACKGROUND

The outbreak of the COVID-19 is a recent occurrence: it was originally identified in Wuhan, China, in December 2019, when serious cases of pneumonia of unknown etiology were first reported. Caused by the SARS-CoV-2 virus, this illness is characterized by its fast person-to-person spread, as well as its manifestations, including fever, rhinorrhea, sore throat and difficulty breathing found in its symptomatic patients, who account for less than 50% of the total COVID-19 cases whilein most patients, the illness is asymptomatic, which is a key factor in the spreading of the virus and represents an epidemiological challenge in terms of its control (WHO Coronavirus disease (COVID-2019) situation reports at www.who.int/emergencies/diseases/novel-coronavirus-2019/situation-reports ( consulted on August 13, 2020).

Other coronaviruses similar to SARS-CoV-2, known as MERS and SARS, have already caused epidemics in previous decades. SARS shows greater homology with SARS-CoV-2. One of the main similarities between them is that both viruses use the ACE2 protein as a receptor to penetrate human cells. Therefore, in both SARS and SARS-CoV-2, the interaction between the receptor binding domain (RBD) of the S1 viral protein and the ACE2 (*angiotensin-converting enzyme 2*) protein is a decisive factor viral infection. (Walls A et al (2020) Cell https://doi.org/10.1016/j.cell.2020.02.058). The RBD of the S protein of the SARS-CoV-2 is a fragment of approximately 195 amino acids (sequence 333-527), which contains the receptor binding motive (RBM) and is the region in which the virus interacts with the ACE2 receptor.The presence of anti-RBD antibodies correlates with the neutralizing activity found in sera of COVID-19 convalescents (Dai L. and associates; 2020 Cell https://doi.org/10.1016/i.cell.2020.06.035; Quinlan B.D. and associates; 2020 https://doi.orq/10.1101/2020.04.10.036418; Zang J. and associates 2020 at https://doi.org/10.1101/2020.05.21.107565) and https://clinicaltrials.gov/ct2/show/NCT04466085?term=NCT0RR66085&draw=2&rank=1, consulted on August 17, 2020). Therefore, vaccinesthat induce antibodies against the RBD are likely to induce virus neutralizing antibodies.

RBD has been used as specific antigen in vaccines against SARS, in combination with aluminum and other known adjuvants. This strategy is currently applied to the preparation of SARS-CoV-2 vaccines, triggering a highly specific antibody response to the RBDthat is capable to protect against viral cell penetration.

Todate (August 10, 2020), 139 candidate vaccines against the SARS-CoV-2 are in preclinical studiesand 28 candidates are in in clinical trials; at least eight of these candidates (including two in clinical trials) are based on RBD as specific antigen. In addition, three vaccine candidates in preclinical studieshave incorporated some OMVs as vaccine platform (DRAFT landscape of COVID-19 candidate vaccines - August 10, 2020 at https://www.vrvho.int/publications/m/item/draft-landscape-of-covid-19-candidate-vaccines, consulted on August 10, 2020).

The RBD in its monomeric form absorbed on aluminahas also been used on animal experiments, demonstrating that it can induce neutralizing antibodies without antibody-dependent enhancement (Quinlan B.D. and associates 2020 at https://doi.orq/10.1101/2020.04.10.036418; Zang J. and associates (2020) at https://doi.orq/10.1101/2020.05.21.107565). RBD has also been produced in different hosts; the nature of glycosylation in asparagine 331 and 343 depends on the expression host (Chen W.H. and associates 2017 Journal of Pharmaceutical Sciences 106: 1961-1970). The RBD monomer absorbed on Al(OH)₃in concentrations of up to 50 micrograms has been recommended as vaccine against the SARS-CoV in humans (https://clinicaltrials.gov/ct2/show/NCT04466085?term=NCT04466085&draw =2&rank=1, consulted on August 17, 2020). Other formulations have incorporated the RBD protein, including the amino acid residues from 319 to 545, expressed in insect cells and Baculoviridae, but the production of a dimer has not been referenced (Yang J. and associates 2020: Nature, at https://doi.org/10.1038/s41586-020-2599-8).

The technical solution closest to this invention is the COVID-19 vaccine formulation that combines an RBDdimer expressed as tandem in CHO cells, and an adjuvant. This vaccine was developed by the Institute of Microbiology of the Chinese Academy of Sciences, in partnership with Anhui Zhifei Longcom Biopharmaceutics. Their formulation produced a strong immune response, with specific antibody titers of 10⁵ (Dai L. and associates 2020; Cell https://doi.org/10.1016/i.cell.2020.06.035). That candidate vaccine is in clinical trials. In the said invention, the dimer was obtained by cloning the dimer coding sequence; that is, unlike the invention discussed hereunder, that is based on cloning the monomer amino acid sequence that ensuresthe dimerization of the RBD's monomer.

Other vaccine candidates currently in preclinical studiesare based on purified,genetically modifiedvesicles carrying viral fragments. This is the case of the Quadram Institute and BiOMViSSrl (at https://quadram.ac.uk/quadram-researchers-working-on-covid-19-vaccine-join-who-expert-groups, consulted on August 17, 2020). Other companies, such as Intravacc, are combining the immunogenic properties of OMV's with protein S of the SARS-CoV-2 (https://www.hospimedica.com/epivax-and-intravacc-to-jointly-develop-covid-19-vaccine-based-on-novel-click-on-omv-technology/articles/294782783/ epivax-and-intravacc-to-iointly-develop-covid-19-vaccine-based-on-novel-click-on-omv-technology.html, consulted on August 17, 2020).

By combining OMV's of the meningococcal group B bacteria with RBD in the form of a stable dimer, the authors of the present invention unexpectedly found that their candidate was superior to reported vaccine compositions that use RBD, in regards to:antibody production kinetics and intensity of immunological response, affinity of produced antibodies and their neutralizing capacity, and T-Helper 1 ("Th-1") cell response. These are crucial characteristics amidst the SARS-CoV-2 pandemic. The vaccine compositions described in the current invention elicit an anti-RBD IgG antibody response on day 7 after immunization with a polarization of cellular response to a Th1 pattern, characterized by the induction of IFNyand an IgG2a isotype; therefore, the immunopathological effects reported for coronavirus vaccines that induce a Th2 pattern are not observed in this case.

None of the technical solutions and/or scientific publications that preceded this invention had described any vaccine based on a combination between dimerizedRBD and OMV's of the meningococcal group B bacteria. This combination has the remarkable property that the induced immune response to RBD is stronger than the response induced by vaccines that do not contain these vesicles. Therefore, the novelty of this invention concerns new COVID-19 vaccine compositions that induce a strong immune response against the virus.

### Brief description of the invention

The present invention provides vaccine compositions that induce a protective immune response against the SARS-CoV-2 virus that comprise a dimer of the Receptor Binding Domain ("RBD") of the S protein of the SARS-Cov-2 virus, as antigen, as well as outer membrane vesicles ("OMV") of the *Neisseria meningitidis* group B bacteria, as immunopotentiator. Particularly, the RBD antigen comprises residues 319-541, as shown in SEQ ID NO. 1. There is a free cysteine in position 538, which allows the formation of the dimeric structure of RBD. These vaccine compositions may also comprise an adjuvant selected from the group comprising any mineral salt such as aluminum hydroxide, aluminum phosphate and calcium phosphate, among others.

Another embodiment of this invention provides a procedure for the production of OMV consisting of the following steps: i) the OMV is solubilized through agitation in pharmaceutical grade water; and ii) the OMV is then sonicated at a temperature below 10° C.

In another embodiment, this invention refers to the application of the above-mentioned vaccine compositions for the prevention of infection with the SARS-CoV-2 virus; in particular, the application of said vaccine compositions to induce an early IgG antibody response to the SARS-CoV-2 virus.The vaccine composition has an RBD concentration range of 5-50 µg per dose, an OMV concentration range of 10-100 µg and adjuvant concentration range between 0.5 and 2.0 mg/mL, applied in a 1-3 intramuscular injection, preferably 2-injections.

In another specific embodiment, this invention deals with the use of the vaccine compositions defined herein to induce a cellular response against the SARS-CoV-2 virus that is biased to a TH-1 pattern, in an intramuscular immunization scheme of preferably two doses, containing 5-50 µg RBD and 10-100 µg OMV, and aluminum hydroxide 0.5-2.0 mg/mL a 1-3 injection or preferably 2-injections.

### Detailed description of the invention

**Method for obtaining the RBD antigen**The sequence coding the RBD protein is synthetized and sub-cloned in the pcDNA-3.1 expression vector. The RBD aminoacid sequence is region 319-541. The construct containing the target protein is transfected into CHO cells.

The target protein is harvested through centrifugation and filtration. The harvested material is then loaded on a Ni-Sepharose affinity column, followed by purification in a Superdex 200 column. Using appropriate methods, the purified protein is analyzed (molecular size, purity, identity, and aminoacid sequenceusingtechniques as SDS-PAGE, SEC-HPLC and/or MS).

In the course of the purification of RBD in the presence of air (i.e., mild oxidizing conditions), RBD is dimerized through adisulfide bridge linking the two free cysteine at position 538 in both monomers. This intermolecular disulfide bride is stable and can only be broken under reductive conditions.

The OMV's are extracted from *Neisseria meningitidis* and purified in accordance with CU 21888A1.

### Vaccine compositions

This formulation is an injectable suspension that contains the RBD dimer, outer membrane vesicles (OMVs) of *Neisseria meningitides* group B bacteria, and an aluminum hydroxide as adjuvant.

The newly developed vaccines discussed herein contain, as relevant antigen, the RBD of protein S of the SARS-CoV-2 virus, which is expressed as a stable dimer. The RBDamino acid sequence is region 319- 541; there are nine cysteine residues in this region, eight of which form four intramolecular disulfide bridges creating a stabledomain structure. In the native protein cysteine 538 ("C 538") forms an intramolecular disulfide bridge. In this construct, C538 is free, as a consequence of the sequence truncated in residue 541. In consequence, an intermolecular disulfide bridge may be formed between two free C 538, generatingan stable dimer that is more immunogenic than the corresponding monomer.

The outer membrane vesicle (OMV) of the meningococcal B bacteria, (150-300 nm), is a key component for the expected effects of this invention. In solution, OMV usually form clusters, with a broad range of particle sizes. The preparation of OMV is a critical process, yielding reproducible immunogenic clusters with the specified size range (WO 2006/008504 A1). This invention supplies OMV preparations that remain stable for at least ten days at temperatures of up to 10° C, preferably 2- 8° C, withparticle sizes of up to 500 nm, preferably 100-300 nm or 150-300 nm, and the invention supplies a procedure for OMV production. For the purpose of stabilizing the OMV's, this invention relies on OMV precipitated in 70% ethanol, which are solubilized in pharmaceutical grade water though agitation at 500 rpm, or preferably at 100-300 rpm, for at least three hours, or preferably one-two hours at 10-25° C. This process is followed by a de-clustering step in an ultrasonic bath at a frequency range between 20 and 40 kHz and a temperature below 10° C, or preferably 2-8° C, for a period between 30 minutes and 4 hours, or preferably 1-3 hours. The rate of OMV de-clustering is measured using Dynamic Light Scattering (DLS) that determines the average size and size distribution of the particles in the preparation.

The two components, the S protein RBD of the SARS-CoV-2 virus, which is expressed as stable dimer, and the OMV's, are blended together through agitation for 30 minutes, or preferably 10-30 min, at an ambient temperature between 18° C and 25° C. This RBD-OMV mix is adsorbed in aluminum hydroxide gel at 70-100 %, through agitation below 500 rpm, preferably 100-300 rpm, for a period between thirty minutes and one hour. Vaccines contain pH-regulating pharmaceutical excipients, including -but not limited to- phosphate buffers in concentration 0.5-1.0 mM, isotonic solutions, including- but not limited to- sodium chloride in concentration 50-150mM, and preservatives, including- but not limited to-thiomersal. The vaccinescovered by this invention are superior to reported vaccines using protein S RBD of the SARS-CoV-2 virus as antigen in three features that are particularly critical in the context of the current pandemic: antigen production kinetics and intensity; antibody affinity and virus neutralizing capacity; and TH-1 cellular response.

### Routes of administration

The SARS-CoV-2 vaccines based on an RBD dimer and meningococcal B OMV's are administered through intramuscular or subcutaneous shots at an RBD dose between 5-50 µg, preferably, 10-20 µg and OMV dose between 20-50 µg, in a treatment of two injections administered at 21-28-days interval. These vaccines may contain a mineral salt, including- but not limited to- aluminum hydroxide, aluminum phosphate and calcium phosphate, in concentrations between 500-2 500 µg, preferably 500-1000 µg.

These formulations are administered following a schedule of one to three doses, every 21 to 28 days.

### Description of drawings

Figure 1 - Ingredients of vaccine formulations: A) aminoacid sequence of S protein RBD:residues 319-541; B) Particle size of de-clustered OMV's as determined by DLS; and C) Titers of anti-RBD IgG antibodies induced in BALB/c mice immunized with two batches of RBD formulated in OMV's/Al(OH)₃produced under good manufacturing practice (GMP).
Figure 2 - Titers of anti-RBD IgG antibodies induced seven days after immunization with the RBDdimer formulated in OMV's/Al(OH)₃or in Al(OH)₃. Letters represent statistical differences(p≤0.5).
Figure 3 - Titers of anti-RBD IgG antibodies seven days after immunization with the RBD dimer formulated in OMV's/Al(OH)₃, compared to RBD monomer formulated in Al(OH)₃. Letters represent statistical differences (p≤0.5).
Figure 4 - Kinetics of anti-RBD IgG antibodies induced by different vaccine compositions of the RBD dimer formulated in OMV's/Al/(OH)₃, compared to RBD monomer formulated in Al/(OH)₃ Letters represent statistical differences (p≤0.5).
Figure 5 - Avidity index (%) of RBD antibodies induced by the RBD dimer formulated in OMV's/Al/(OH)₃, compared to RBDmonomer formulated in OMV's/Al/(OH)₃.
Figure 6 -Inhibition of RBD-ACE2 interactionby antibodies induced in BALB/c mice immunized with the RBD dimer formulated in OMV's/Al(OH)₃.
Figure 7 - A) Production of IFNy and IL-4(pg/mL) by spleen cells in mice immunized with RBD dimer in OMV's/Al(OH)₃, and in-vitro re-stimulated with RBD, as determined by quantitative ELISA.

Letters represent statistical differences (p≤0.5), according to the Tukey's test; B) IgG2a/IgG1 isotype ratio as an indication of the induction of a TH-1 pattern.

### Examples of embodiments

### Example 1 - Formulations of the SARS-CoV-2 vaccines

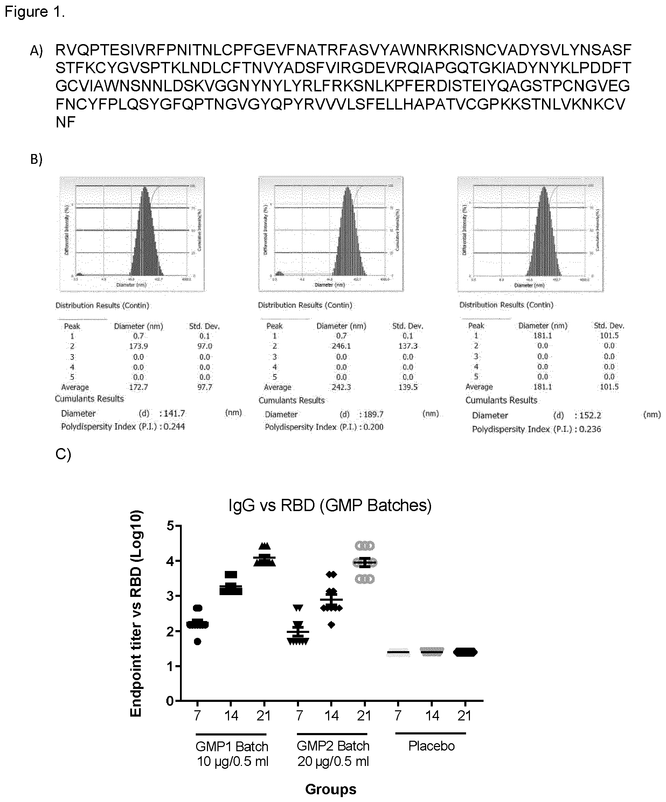

Two formulations were obtained containing: the RBD dimer as antigen and with the sequence shown in Figure 1A, at a concentration of 10-20 µg, de-clustered OMV's with particles between 150 and 300 nm and at a concentration of 20-40 µg, and aluminum hydroxide as adjuvant at a concentration of 500 µg.

The OMV's are precipitated in ethanol and extracted as described in CU 21888A1. The mass of OMV precipitate is weighed, homogenized in water and fully dissolved through mechanical agitation (for aboutone hour) at ambient temperature. Once the OMV's have been dissolved, the solution is checked for the absence of particles and/or turbidity, followed by a de-clustering process in an ultrasonic bath, at ≤10° C during the sonication process. At the end of de-clustering, samples are taken to determine the OMV particle sizes byDLS (Figure 1B). Particles with sizes 150-300 nm will operate as controls for this process. The de-clustered OMV's are stored at 2°-8° C.

The mix of RBD and de-clustered OMV's (OMV-RBD) is homogenized through mild agitation (at 100-300 rpm) for 15 minutes.

Finally, OMV-RBD is mixed with aluminum hydroxide and the formulations are filtered, while the aforementioned parametersare maintained for 0.5-1 hour. The formulation is stored at 2°-8° C. The formulations of RBD dimer in OMV/Al(OH)₃(Figure 1C) produced in accordance to good manufacturing practices (Figure 1C), induce an anti-RBD IgG antibody response in BALB/c mice, stronger than the obtained in animals treated with placebo (without the vaccine antigen).

### Example 2 - Unlike the formulation of RBD dimer in Al(OH)₃, the formulation of RBD dimer in OMV/Al(OH)₃induces an early antibody response in BALB/c mice.

BALB/c mice were immunized intramuscularly at time zero with 0.1 mL of one of the following formulations:
- Group 1:10 µg of RBD dimer blended with 20 µg of un-clustered OMV, co-absorbed in 800 µg of Al(OH)₃;
- Group 2:10 µg of RBD dimer, co-absorbed in 800 uµ of Al(OH)₃;
- Group 3:20 µg of un-clustered OMV absorbed in 800 uµ of Al(OH)₃and
- Group 4: 800 µg of Al(OH)₃.

Groups 3 and 4 operate as negative controls.

Blood is drawn at time zero and day 7 after immunization. Serum from the immunized animals is tested in an indirect ELISA to determine anti-RBD antibody titers. The Nunc MaxiSorp^{™} high protein-binding capacity 96-wells ELISA plates were coated with 50µL of RBD at a concentration of 3 µg/mL in a carbonate-bicarbonate coating buffer,pH 9.6, and incubatedfor one hour at 37° C. The plates were washed three times using a cleaning solution. The non-coated sites were blocked using 100 µL of a 5% skimmed milk blocking solution for one hour at 37° C, followed by another wash step, as described above. Sera serial dilutions inphosphate buffer, pH 7.2, 1% BSA, were added in (1:3), generally from a baseline of 1/50 and 50 µL/well. The plates were incubated for 1 hour at 37°C and washed again. Next, 50 µL of a dilution of anti-mouse IgG conjugated to peroxidase was added in a regulatory phosphate buffer solution (pH 7.2), 1% BSA (1:5000) and incubated for one hour. After one last washing, the peroxidase enzyme substrate solution (50 µL/well) was applied. It was then incubated in the dark for 20 minutes and the reaction was stopped with 50 µL/well of 2N H2SO4. Absorbance was read at 450 nm in a Multiskan EX ELISA reader (Thermo Scientific). The IgG titer was defined as the inverse of the serum dilution of each individual animal serum reaching four times the value of the mean absorbance of pre-immune sera (T0) at a 1:50 dilution. For the analysis and presentation of the results, the Log10 of the titer for each individual animal was calculated. To define respondent animals, a log titer >1.70 was taken as the cutoff value, corresponding to a > 1:50 serum dilution. A titer value equal to25 and a Iog10 of 1.4 were set for the animals whose titer was lower than the assay detection limit.

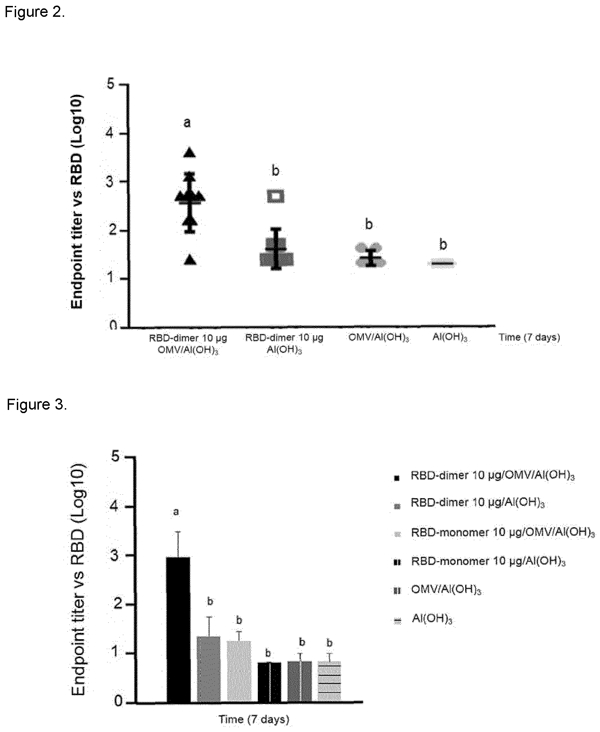

Figure 2 shows the early IgG antibody response induced by Group 1 formulation, which contains theRBD dimer in OMV/Al/(OH)₃, and Group 2 formulation, which containsthe RBD dimer in Al/(OH)₃. Seven days after immunization, the induced RBD antibody response is significantly higher (p≤0,05, one-way ANOVA test and Tukey's multiple comparison test) in 9 out of 10 mice immunized with the formulation containing the RBD dimer in OMV/Al(OH)₃, as compared with those immunized with the formulation containing the RBD dimer in Al(OH)₃. This difference is attributable to the immunopotentiating capacity of the OMV's present in Group 1 formulation.

### Example 3 - Unlike the formulation of an RBD monomer in OMV/Al(OH)₃, the formulation of theRBD dimer in OMV/Al(OH)₃ induces an early antibody response in BALB/c mice.

BALB/c mice were immunized intramuscularly at time zero with 0.1 mL of one of the following formulations:
- Group 1:10 µg of RBD dimer blended with 20 µg of un-clustered OMV, co-absorbed in 800 uµ of Al(OH)₃;
- Group 2:10 µg of RBD dimer, co-absorbed in 800 µg of Al(OH)₃;
- Group 3:10 µg of RBD monomer, blended with 20 µg of un-clustered OMV, absorbed in 800 µg of Al(OH)₃;
- Group 4:10 µg of RBD monomer absorbed in 800 µg of Al(OH)₃;
- Group 5:20 µg of un-clustered OMV's absorbed in 800 µg of Al(OH)₃ and
- Group 6: 800 µg of AI(OH)3.

Groups 5 and 6 operate as negative controls.

The procedure for blood collection and serum evaluation are the same as in Example 2.

Figure 3 shows the early IgG antibody response induced by formulations that contain RBD dimer in OMV/Al/(OH)₃, as compared with formulations that contain an RBD monomer in the same mix. Seven days after immunization, the RBD antibody response is significantly higher (p≤0,05, one-way ANOVA and Tukey's multiple comparison test) in the mice that received the Group-1 formulation containing RBD dimer in OMV/Al(OH)₃, as compared with the Group-3 formulation that contains an RBD monomer in the same mix. This property is attributable to the stronger immunogenicity of the dimer versus the monomer.

**Example 3** - **After two doses, the RBD dimerformulated inOMV/Al(OH)₃ is highly immunogenic in mice.**BALB/c mice were immunized intramuscularly with two vaccine doses; i.e., at time zero and 14 days later, with 0.1 mL of one of the following formulations:
- Group 1: 3 µg of RBD dimer blended with 20 uµ of un-clustered OMV, co-absorbed in 800 µg of Al(OH)₃;
- Group 2:10 µg of RBD dimer, blended with 20 uµ of un-clustered OMV's, co-absorbed in 800 µg of Al(OH)₃;
- Group 3:3 µg of RBD dimer, absorbed in 800 uµ of Al(OH)₃;
- Group 4:10 µg of RBD dimer, absorbed in 800 uµ of Al(OH)₃;
- Group 5:20 µg of un-clustered OMV's, absorbed in 800 uµ of Al(OH)₃,
- Group 6: 800 µg of Al(OH)₃.

Groups 5 and 6 are negative controls.

Blood is drawn for serum at 0, 7, 14, 21 and 28 days; in other words, pre-immune serum, and 7 and 14 days after each injection. The purpose of this immunization schedule is twofold: to compare the doses of 3 and 10 µg of RBD dimer in a formulation with OMV/Al(OH) ₃and with Al/(OH)₃, and to assess the antibody kinetics induced by these formulations.

Anti-RBD antibodies were evaluated in an ELISA as described in Example 2.

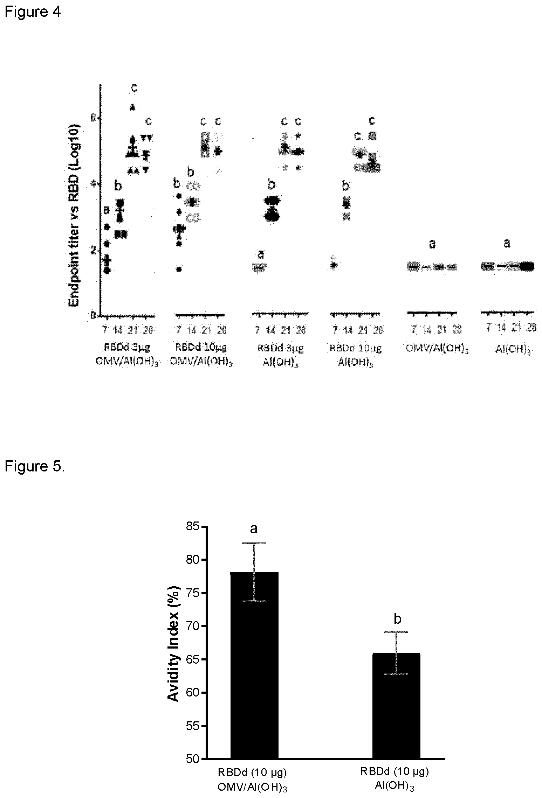

As shown in Figure 4, the formulation containingthe RBD dimer in OMV/Al(OH)₃ triggers a dose-dependent kinetics that increases sharply after the second dose. However, no significant differences are observed between the formulation of the RBD dimer in OMV/Al(OH)₃and the formulation of the RBD dimer in Al(OH) ₃after the second dose of the vaccine. The antibody titers on days 21 and 28 are high for both formulations: 10⁴-10⁶.

### Example 5: Affinity of antibodies induced by the formulation of an RBD dimer in OMV/Al(OH) ₃and the formulation of an RBD dimer in Al(OH)₃

To assess the affinity of the induced antibodies, their avidity was determined by disrupting the antigen-antibody reaction through the addition of the chaotropic agent ammonium thiocyanate (NH₄SCN). The plates were coated with 50µg of RBD (3 µg/mL) in carbonate-bicarbonate coating buffer pH 9.6, and incubated during one hour at 37° C. The plates were then washed three times using a cleaning solution; the non-coated sites were blocked with 100 µL of 5% skimmed milk blocking solution for one hour at 37° C. Sera (at dilutions giving approximately an absorbance value of 1 in an ELISA) were added. The sites were incubated at 37° C for one hour, followed by the addition of 2M NH₄SCN for 15 minutes at ambient temperature. The procedure continued as described in Example 2.

The avidity indicates the percentage of IgG antibodies that remain attached to the antigen after treatment with the chaotropic agent. This avidity is calculated using the following formula: (IgG titer with NH₄SCN/IgG titer without NH₄SCN) *100. Antibodies with an avidity above 50% are considered to have good avidity. The rate of avidity is determined only for respondent animals (titer >1.70), determined in anELISA.

As shown in Figure 5, the antibodies induced by the formulation of RBD in OMV/AI(OH) ₃have higher affinity (p≤0.05) than the antibodies induced by the RBD dimer in AI(OH) ₃, indicating the superior quality of the induced immune response of the formulations covered by this invention.

### Example 6 - Functional activity of the anti-RBD antibodies induced by the RBD dimer formulated in OMV/Al(OH)₃

### RBD-ACE2interaction is inhibited by induced antibodies

The serum drawn at day 28 from mice immunized as described in Example 2 was subjected to an ELISA in order to determine theinhibition of the RBD-ACE2interaction.

The ELISA plates were coated with mouse ACE-Fc (5 µg/mL in a carbonate-bicarbonate buffer pH 9.6) and incubated at 4° C overnight.The plates were then washed three times using a cleaning dissolution. The non-coated sites were blocked using 200 µL of a 2% skimmed milk blocking solution for one hour at 37° C. Human RBD-Fc was prepared (40 ng/mL in, pH 7.2 containing 2% milk). Sera from pre-immune and immunized mice were prepared at dilutions ranging from 1:25 to 1:400. As negative control, an irrelevant protein, human PDL1-F antibody, was used.

The diluted sera were pre-incubated with human RBD-Fc at a 1:1 ratio at 37° C for 1 h, applied to the plate(50 µL/well),incubated at 37° C for 1.30 h andwashed once.

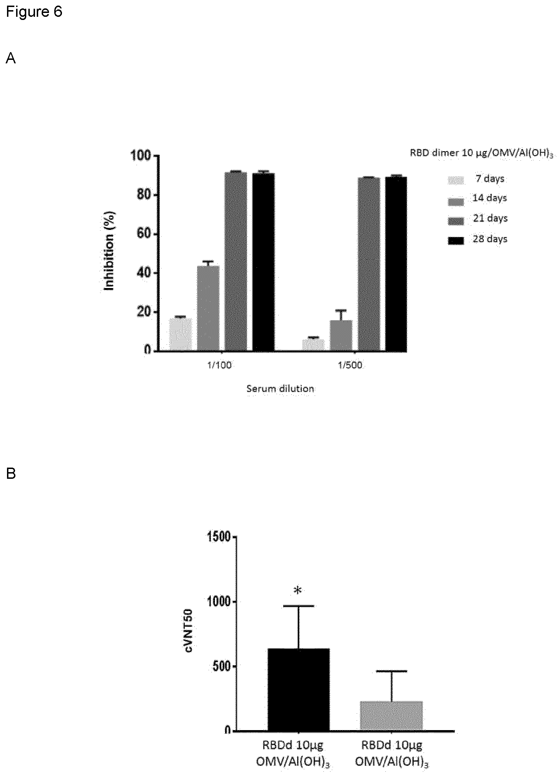

After another washing step, 50 µL of human IgG antibodies conjugated to alkaline phosphatase in a phosphate buffer pH 7.2 containing 2% milk(1:1000) and the plates were incubated at 37° C for 1 h. After one last wash step, PNPP (1mg/mL) in diethanolamine buffer was added (50 µL/well). The plate was incubated in the dark for 20 min and the reaction was stopped by adding NaOH 3M (50µL/well). Absorbance at 405 nm was read using an ELISA reader.The inhibition was calculated using the following formula: (1-Abs405nm human RBD Fc + mouse serum/Abs405nm human RBD Fc) *100.Figure 6A illustrate the capacity to inhibit RBD-ACE2 interaction of the serum extracted from mice immunized with the formulation described in this invention.

### Neutralizing capacity of the anti-RBD antibodies elicited against the SARS-CoV-2 virus

The SARS-CoV-2-neutralizing capacity of the serum obtained from mice that had been immunized following the schedule described in Example 2 was assessed in a colorimetric assay using Neutral Red. Vero E6 cells were cultivated in plates in MEM+ 2% fetal bovine serum, 25 mM/mL L-glutamine, 2 µg/mL bicarbonate, 80 µg/mL gentamicin, and 5 µg/mL amphotericin B. The supernatant was removed, and 100 µL of PBSpH-7.2 containing 0.02% Neutral Redwas added to each well. The plates were incubated at ambient temperature for 1 h. The Neutral Red solution was discarded. The cell monolayer was washed twice with PBS, 0.05% Tween 20. To each well was added 100 µL of lysis solution (50 parts of absolute ethanol, 49 parts of ultrapure water and one part of glacial acetic acid). The plate was incubated at ambient temperature for 15 min and the absorbance was measured at 540 nm. The highest dilution of the serum with an optical density value above the cut-off value, was considered the neutralizing titer. The cut-off value was calculated as the half value of the average optical density of the cell-coated control wells. The serum neutralizing capacity induced by a formulation of RBD dimer in OMV/AI(OH) ₃was demonstrated in a viral neutralization assay. It reaches a neutralizing titer of 1:640, on average, on day 28 after immunization, as illustrated in Figure 6B.

### Example 7. TH-1 pattern of the cellular immune response, as determined by induced IFNγ and the IgG2a/ lgG1 ratio

The cellular immune response of mice that had been previously inoculated following the scheduledescribed in Example 2 was assessed in sera extracted on day 28. The splenocytes of BALB/c mice immunized with a formulation of RBD dimer in OMV/Al(OH)₃or a formulation of RBD dimer in Al(OH)₃ were isolated and stimulated *in-vivo* in the presence of RBD (5 µg/mL). The cell concentration was 1 × 10⁶ cells/mL. IL-4 and IFNy were determined in the culture supernatant using a quantitative ELISA, 72 hours after stimulation.

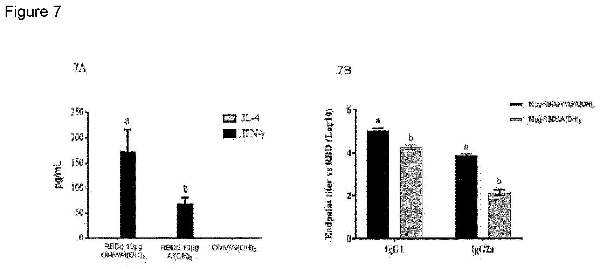

As illustrated in Figure 7A, the RBD dimer in OMV/Al(OH)₃ induces higher levels of IFNy than the RBD dimer in Al/(OH)₃. The T-cell response was biased to a TH-1 pattern. The IgG response The IgG2a/IgG1 ratio is higherin the RBD dimer in OMV/Al(OH)₃ than in RBD dimer in Al/(OH)₃; evidencing a response bias favoring the TH-1 pattern.

## Claims

1. A vaccine composition that induces an immune response against the SARS-CoV-2 virus**characterized by** a portion of the receptor binding domain of the S protein of the SARS-CoV-2 virus, as antigen, outer-membrane vesicles of the *Neisseria meningitidis* Group B bacteria (OMVs), as immunopotentiating component, and an adjuvant.

2. The vaccine composition according to -claim 1 where the antigen has the sequence SEQ ID NO. 1.

3. The vaccine composition according to any of the claims 1-2 wherein the RBD is in the form of a dimer.

4. The vaccine composition according to claim 3 wherein the dimer is formed through disulphide bridges between two free cysteine (C538).

5. The vaccine composition under claim 1 where the adjuvant is selected from the following group:
- Aluminum hydroxide;
- Aluminum phosphate; and
- Calcium phosphate

6. The vaccine composition according to claim 1 wherein the outer-membrane vesicles (OMV's) of the *Neisseria meningitidis* group B used as immunopotentiating component, has a particle size in a range between 150-300 nm.

7. The vaccine composition according to claim 1 wherein dose range of the RBD is from 5 to 50 µg.

8. The vaccine composition according to claim 1 that also comprises pharmaceutically suitable excipients.

9. A procedure for obtaining the OMV's of claim 6 that comprises the following stages:
a) The OMV's are solubilized in pharmaceutical grade water by agitation; and
b) The OMV's are sonicated at a temperature below 10° C.

10. Use of the vaccine composition of any of the claims 1 to 8 for preventing the infection with SARS-CoV-2 virus.

11. Use of the vaccine composition of any of the claims 1 to 8 to prevent the infection with SARS-CoV-2 virus, where elicited specific antibodies against the virus are needed seven days after immunization.

12. Use of the vaccine composition of any of the claims 1 to 8 to prevent the infection with SARS-CoV-2 virus, where a neutralizing antibody response and a Th-1-pattern cellular response against the virus are required after the administration of two doses.

13. Use of the vaccine composition of any of the claims 1 to 8 to induce an antibody response against the SARS-CoV-2 virus by intramuscular route of RBD in a dose range from 5 to 50 µg and *N*. *meningitidis* OMV's in a dose range from 10 to100 µg in an immunization schedule of one to three doses.
